Europäisches Patentamt

European Patent Office

Office européen des brevets

(11) Publication number: **0 154 472**
**B1**

(12) **EUROPEAN PATENT SPECIFICATION**

(45) Date of publication of patent specification: **11.10.89**

(51) Int. Cl.⁴: **C 12 P 21/02**

(21) Application number: **85301229.2**

(22) Date of filing: **25.02.85**

(54) **Process for the production of L-aspartyl-L-phenylalanine ester.**

(30) Priority: **07.03.84 JP 43489/84**
**27.07.84 JP 156686/84**

(43) Date of publication of application:
**11.09.85 Bulletin 85/37**

(45) Publication of the grant of the patent:
**11.10.89 Bulletin 89/41**

(84) Designated Contracting States:
**CH DE FR GB IT LI NL**

(56) References cited:
**EP-A-0 074 095**
**EP-A-0 124 313**
**DD-A- 141 323**
**US-A-4 390 629**

**FERMENTATION TECHNOLOGY TODAY,**
**PROCEEDINGS OF THE 4th INTERNATIONAL**
**FERMENTATION SYMPOSIUM, 19th-25th**
**March 1972, Kyoto, Japan, pages 279-272,**
**Society of Fermentation Technology, JP; T.**
**HATA et al.: "Yeast proteinase"**

(73) Proprietor: **AJINOMOTO CO., INC.**
**5-8, Kyobashi 1-chome, Chuo-ku**
**Tokyo 104 (JP)**

(72) Inventor: **Yokozeki, Kenzo**
**No.1523-4, Shinsaku Takatsu-ku**
**Kawasaki-shi Kanagawa-ken (JP)**
Inventor: **Hirose, Yoshiteru**
**No.1982-106, Fueda**
**Kamakura-shi Kanagawa-ken (JP)**
Inventor: **Majima, Eiji**
**No.2-20-8, Kannon Kawasaki-ku**
**Kawasaki-shi Kanagawa-ken (JP)**
Inventor: **Sato, Takeru**
**No.2-20-8, Kannon Kawasaki-ku**
**Kawasaki-shi Kanagawa-ken (JP)**
Inventor: **Yukawa, Toshihide**
**No.50-40, Tana-cho Midori-ku**
**Yokohama-shi Kanagawa-ken (JP)**

(74) Representative: **Armitage, Ian Michael et al**
**MEWBURN ELLIS & CO. 2/3 Cursitor Street**
**London EC4A 1BQ (GB)**

# EP 0 154 472 B1

(56) References cited:
CHEMICAL ABSTRACTS, vol. 81, no. 9, 2nd September 1974, p. 309, no. 48563k, Columbus, OH (US)

CHEMICAL ABSTRACTS, vol. 78, no. 15, 16th April 1973, p. 318, no. 95705f, Columbus, OH (US); H.E.MUELLER et al.: "Proteolytic activity of Cryptococcus neoformans against human plasma proteins

CHEMICAL ABSTRACTS, vol. 85, 1976, pp. 239-240, no. 139540z, Columbus, OH (US); M.GUEGUEN et al.: "Characteristics of the proteolytic system of Geotrichum candidum"

## Description

Background of the invention
(Field of the invention)
This invention relates to a process for producing L-aspartyl-L-phenylalanine esters.

(Description of the prior art)
L-aspartyl-L-phenylalanine methyl ester (abbreviated as APM hereinafter) is a peptide which has become noted as a sweetener in recent years.

It is well-known that APM can be produced by chemical synthesis or enzymatic synthesis.

A chemical synthesizing process for the production of APM comprises condensing N-protected L-aspartic acid anhydride and L-phenylalanine methyl ester (abbreviated as PM hereinafter) to obtain N-protected APM and then removing the protective group afterwards. An enzymatic synthesizing process comprises exerting the effect of a protein-decomposing enzyme on N-protected L-aspartic acid and PM to obtain N-protected APM or the PM adduct of N-protected APM and then removing the protective group to form APM. However, both processes require the complicated steps of introducing the protective groups and removing the same.

There is also known a process for producing APM without using the protective groups (Japanese Patent Kokai No. 126796/1983, "Digests of the Publications at the Annual Meeting of the Agricultural Chemical Society of Japan" in 1983, P.42) which is a microbiological synthetic process using one of Pseudomonas, Torulopsis, Rhodotorula, and Sporobolomyces, but this is not always suitable for the industrial production of APM because of the extremely low yields.

The present inventors have previously found that the employment of a microorganism can bring about the direct and effective formation of APM from L-aspartic acid and PM (Japanese Patent Application No. 75559/1983).

However, a difficult aspect of a process for producing APM using L-aspartic acid without the protective groups is that the reaction of L-aspartic acid and PM to form APM is an equilibrium reaction, and the equilibrium prevents the substrates from being converted efficiently to APM.

Summary of the invention
The present inventors have found that L-aspartyl-L-phenylalanine R-ester (hereinafter abbreviated to APR), wherein R-ester is an ester residue of an alcohol of 2 or more carbon atoms or a substituted or unsubstituted phenol, can be efficiently formed by the action of a microorganism on L-aspartic acid or a salt thereof and L-phenylalanine R-ester of acid salt thereof (abbreviated to PR hereinafter) since the APR obtained is removed from the reaction system because of its low solubility. L-aspartic acid can be used as the free acid or as its acid salt (e.g., hydrochloride) or alkaline salt (e.g. sodium).

APR itself can be used as a sweetener or as a raw material to synthesize APM by ester exchange (or any other methods).

Accordingly, this invention is directed to a process for the production of APR by the condensation of L-aspartic acid and PR under the action of an effective microorganism chosen from Corynebacterium, Candida, Escherichia, Flavobacterium, Geotrichum, Micrococcus, Pachysolen, Trichosporon, Xanthomonas, Kluyveromyces, Endomyces, Arthrobacter, Cellulomonas and Brevibacterium.

The process can be carried out by contacting L-aspartic acid and PR with microorganism cells, culture solutions or enzymic materials obtained by treating the above-mentioned microorganisms.

Detailed description of the preferred embodiments
The following are examples of the microorganisms which have the ability to change L-aspartic acid and PR to APR by the condensation process of this invention:

| | |
|---|---|
| Corynebacterium SP | ATCC 21251 |
| Corynebacterium xerosis | ATCC 373 |
| Candida intermedia | FERM-BP508 |
| Escherichia coli | FERM-BP477 |
| Flavobacterium sewanens | FERM-BP476 |
| Geotrichum candidum | IFO 4599 |
| Micrococcus luteus | ATCC 4698 |
| Pacysolen tannophilus | IFO 1007 |
| Trichosporen capitatum | IFO 1197 |
| Xanthomonas campestris | FERM-BP507 |
| Kluyveromyces thermotolerans | IFO 0662 |
| Endomyces ovetencis | IFO 1201 |
| Arthrobacter citreus | ATCC 11624 |
| Callulomonas flavigena | ATCC 8183 |
| Brevibacterium linens | ATCC 8377 |

The cells of these microorganisms can be obtained by using ordinary culture media. Further, L-aspartic acid and PR may be added at the beginning or during the process of cultivation of the cells.

The culture media to be used for the microorganisms are ordinary ones containing usual carbon and nitrogen sources and inorganic ions in addition to L-aspartic acid and PR. Moreover, the addition of trace amounts of organic nutritive substances such as vitamins and amino acid often brings about desirable results.

The carbon sources suitable for use herein include carbohydrates such as glucose and sucrose, organic acids such as acetic acid, and alcohols. The nitrogen sources suitable for use herein include ammonia gas, aqueous ammonia and ammonium salts. The inorganic ions are suitably selected from e.g. magnesium ion, phosphoric acid ion, potassium ion and iron ion, as necessary.

The cultures are suitably conducted under aerobic conditions at pH 4—8 at suitable temperatures controlled within the range of 25—45°C and for 1—10 days.

The microorganisms which can be used in this invention include the whole culture solutions obtained after completion of cultivation, microorganisms separated from the culture solutions, or washed microorganisms. Also, the microorganisms which can be used may be freeze-dried, acetone-dried, contacted with toluene, surfactants, etc., treated with lysozyme, exposed to ultrasonic waves or mechanically ground, and enzyme protein fractions obtained from these cell-treating materials having enzymic activity capable of changing L-aspartic acid and PR to APR. Fixed cells of these microorganisms, insolubilized materials of treated cells, etc. may be used.

As aqueous media, there can be used those containing water, buffers, and organic solvents such as ethanol. Moreover, nutritive elements needed for the growth of microorganisms, anti-oxidants, surfactants, coenzymes, hydroxylamine and metallic ions, etc. can be added to the aqueous media if necessary.

When the cells of the above-mentioned microorganisms are grown in aqueous media and simultaneously brought into contact with L-aspartic acid and PR to exert the action thereon, the aqueous media should contain L-aspartic acid, PR, and also nutritive elements as indicated above.

When the whole culture solutions, culture cells or treated cell materials of the above-mentioned microorganisms are brought directly into contact with L-aspartic acid and PR to exert the action thereon, an aqueous medium is prepared by dissolving or suspending L-aspartic acid, PR and the culture solution, culture cells, or treated cell materials, and is controlled suitably at a temperature of 10—70°C and pH 4—8, and allowed to stand for a while or stirred. APR is produced and accumulated in the aqueous medium after 5—100 hours.

The APR thus produced can be separated and purified by the known separation processes. The APR obtained is determined with an amino-acid analyzer.

The invention, having been generally described, will be better understood by reference to the following specific examples which are included herein for purposes of illustration only and are not intended to be limiting.

Example 1

Into 500 ml-flasks was introduced 50 ml each of a medium (pH 7.0) containing 2.0 g/dl of glucose, 0.5 g/dl of $(NH_4)_2SO_4$, 0.1 g/dl of $KH_2PO_4$, 0.1 g/dl of $K_2HPO_4$, 0.1 g/dl of $MgSO_4 \cdot 7H_2O$, 0.05 g/dl of $FeSO_4 \cdot 7H_2O$, 1 mg/dl of $MnSO_4 \cdot 4H_2O$, 1.0 mg/dl of yeast extract, 0.05 g/dl of malt extract, and 4.0 g/dl of calcium carbonate, which was sterilized at 120°C for 15 minutes.

Each one of the thus prepared media was inoculated, using a platinum loop, with Flavobacterium sewanens FERM-BP476 or Arthrobacter citreus ATCC 11624 incubated in a bouillon-agar medium at 30°C for 24 hours, and cultured at 30°C for an additional 20 hours. The cells were harvested from this culture solution by centrifugation, washed once with the same amount of phyiological saline as that of the culture solution and collected.

These cells of the microorganisms were added to Reaction Solution A shown in Table 1 at 5 g/dl (final conditions, pH 5.4, 5 ml) and allowed to react with an amino acid analyzer to give the results in Table 2.

TABLE 1
Reaction Solution A

| | |
|---|---|
| L-aspartic acid | 10 g/dl |
| L-phenylalanine ester or its hydrochloride shown in Table 2 | 15 g/dl |

* The above substrates are contained in 0.1M phosphoric acid buffer (final pH 5.4).

# EP 0 154 472 B1

## TABLE 2

| Phenylalanine ester or its hydrochloride | APR formed : mg/dl | |
| --- | --- | --- |
| | Flavobacterium sewanens FERM-BP476 | Arthrobacter citreus ATCC 11624 |
| Phenylalanine methylester hydrochloride | 590 | 583 |
| Phenylalanine ethylester hydrochloride | 710 | 702 |
| Phenylalanine n-propylester hydrochloride | 812 | 810 |
| Phenylalanine iso-propylester hydrochloride | 1020 | 1005 |
| Phenylalanine n-butylester hydrochloride | 920 | 911 |
| Phenylalanine isobutylester hydrochloride | 880 | 865 |
| Phenylalanine sec-butylester hydrochloride | 850 | 841 |
| Phenylalanine tert.-butylester hydrochloride | 821 | 809 |
| Phenylalanine cyclohexylester hydrochloride | 807 | 799 |
| Phenylalanine amylester hydrochloride | 792 | 785 |
| Phenylalanine hexylester hydrochloride | 774 | 762 |
| Phenylalanine heptylester hydrochloride | 772 | 760 |
| Phenylalanine octylester hydrochloride | 763 | 740 |
| Phenylalanine nonylester hydrochloride | 751 | 729 |
| Phenylalanine decylester hydrochloride | 748 | 715 |
| Phenylalanine benzylester hydrochloride | 729 | 699 |
| Phenylalanine p-nitrophenylester | 703 | 689 |
| Phenylalanine phenylester | 699 | 678 |

## Example 2

Cells of the microorganisms shown in Table 4, and washed in a manner similar to Example 1, were added to Reaction Solution B shown in Table 3 at 5 g/dl (final condition, pH 5.4, 5 ml), and kept at 37°C for 16 hours. The resulting aspartyl phenylalanine isopropylester was determined with an amino acid analyzer to give the results in Table 4.

## TABLE 3
### Reaction Solution B

| | |
| --- | --- |
| L-aspartic acid | 10 g/dl |
| L-phenylalanine isopropylester hydrochloride | 15 g/dl |

The above substrates are contained in 0.1M phosphoric acid buffers (final pH 5.4).

5

# EP 0 154 472 B1

## TABLE 4

| Microorganisms | | Reaction solution aspartyl phenylalanine isopropylester formed (mg/dl) |
|---|---|---|
| Corynebacterium sp. | ATCC 21251 | 862 |
| Corynebacterium xerosis | ATCC 373 | 324 |
| Candida intermedia | FERM-BP508 | 425 |
| Escherichia coli | FERM-BP477 | 1172 |
| Flavobacterium sewanens | FERM-BP476 | 1050 |
| Geotrichum candidum | IFO 4599 | 204 |
| Micrococcus luteus | ATCC 4698 | 915 |
| Pacysolen tannophilus | IFO 1007 | 148 |
| Trichosporen capitatum | IFO 1197 | 163 |
| Xanthomonas campestris | FERM-BP507 | 372 |
| Kluyveromyces thermotolerans | IFO 0662 | 135 |
| Endomyces ovetencis | IFO 1201 | 399 |
| Arthrobacter citreus | ATCC 11624 | 1018 |
| Cellulomonas flavigena | ATCC 8183 | 795 |
| Brevibacterium linens | ATCC 8377 | 939 |

Example 3

Into 100 ml of Reaction Solution B was introduced 5 g of Flavobacterium sewanens FERM-BP476 grown and washed in a manner similar to Example 1, and the reaction was carried out at 37°C for 24 hours.

The resulting reaction solution was spotted on a TLC plate for development in the form of a belt, and developed with a solvent system consisting of n-butanol:acetic acid:water=2:1:1. Part of the product aspartyl phenylalanine isopropylester was taken out and extracted with distilled water. Then, the resulting reaction product was crystallized to obtain 1023 mg of crystals. The obtained crystals were characterized as to optical rotation, melting point, and specific rotatory power, and the product obtained from Reaction Solution B was identical to an authentic aspartyl phenylalanine isopropylester specimen.

Example 4

Into 100 ml of Reaction Solution B was introduced 5 g of Arthrobacter citreus ATCC 11624 grown and washed in a manner similar to Example 1, and the reaction was carried out at 37°C for 24 hours.

The resulting reaction solution was spotted on a TLC plate for development in the form of a belt, and developed with a solvent system consisting of n-butanol:acetic acid:water=2:1:1. Part of the product aspartyl phenylalanine isopropylester was taken out and extracted with distilled water. Then, the resulting reaction product was crystallized to obtain 1005 mg of crystals. The obtained crystals were characterized as to optical rotation, melting point, and specific rotatory power, and the product obtained from Reaction Solution B was identical to an authentic aspartyl phenylalanine isopropylester specimen.

Example 5

Into a solution of Escherichia coli FERM-BP477, cultured at 30°C for 12 hours in the same medium used in Example 1, was poured under sterile conditions 10 ml of an aqueous solution (adjusted to pH 5.4) containing 5 g/dl of L-aspartic acid and 10 g/dl of L-phenylalanine isopropylester, and the cultivation was further continued for 10 hours after the solution was adjusted under sterile conditions to pH 5.4. It was maintained at a pH of 5.4 by adjustments at intervals of 2 hours during incubation.

The resulting product in this culture solution was determined with an amino-acid analyzer and 489 mg/dl of aspartyl phenylalanine isopropylester was formed.

6

**EP 0 154 472 B1**

Example 6

Into the culture solution of Brevibacterium linens ATCC 8377, cultured at 30°C for 12 hours in the same medium used in Example 1, was poured under sterile conditions 10 ml of an aqueous solution (adjusted to pH 5.4) containing 5 g/dl of L-aspartic acid and 10 g/dl of L-phenylalanine isopropylester, and the cultivation was further continued for 10 hours after the solution was adjusted under sterile conditions to pH 5.4. It was maintained at a pH of 5.4 by adjustments at intervals of 2 hours during incubation.

The resulting product in this culture solution was determined with an amino-acid analyzer and 479 mg/dl of aspartyl phenylalanine isopropylester was formed.

Example 7

Flavobacterium sewanens FERM-BP476, grown and washed in a manner similar to Example 1, was added to Reaction Solution A (containing phenylalanine n-butyl ester) at 5 g/dl (final conditions, pH 5.4, 5 ml) and allowed to react at 37°C for 16 hours.

1 liter of the resulting enzyme reaction solution was stood for 24 hours at 0°C and 3 g of precipitated crystals were filtered. Part of the product was taken out and measured by high-speed liquid chromatography (column, silicon 0DS; eluent, methanol-water) and the product was found to contain 1.67 g opf L-aspartyl-L-phenyl-alanine n-butylester. These crystals were added to a solution consisting of 3.8 g of 35% hydrochloric acid, 1.0 g of methanol and 2.0 g of water and agitated for 7 days at 15°C. The resulting product was determined with an amino-acid analyzer, infrared spectrum, acid titration and hydrochloric acid titration to indicate that the product gives 0.38 g of L-aspartyl-L-phenylalanine methylester hydrochloride. The yield with respect to L-aspartyl-phenylalanine butylester is 23%.

Example 8

Arthrobacter citreus ATCC 11624, grown and washed in a manner similar to Example 1, was added to Reaction Solution A (containing phenylalanine n-butyl ester) at 5 g/dl (final conditions, pH 5.4, 5 ml) and allowed to react at 37°C for 16 hours.

1 liter of the resulting enzyme reaction solution was stood for 24 hours at 0°C and 3 g of precipitated crystals were filtered. Part of the product was taken out and measured by high-speed liquid chromatography (column, silicon 0DS; eluent, methanol-water) and the product was found to contain 1.65 g of L-aspartyl-L-phenylalanine n-butylester. These crystals were added to a solution containing 3.8 g of 35% hydrochloric acid, 1.0 g of methanol and 2.0 g of water and agitated for 7 days at 15°C. The product was filtered and dried to obtain 0.39 g of dried white crystals. The resulting product was determined with an amino-acid analyzer, infrared spectrum, acid titration and hydrochloric-acid titration to indicate that the product gives 0.36 g of L-aspartyl-L-phenylalanine methylester hydrochloride. The yield with respect to L-aspartyl-phenylalanine butylester is 22%.

**Claims**

1. A process for the production of a sweetening agent, which comprises contacting in an aqueous medium L-aspartic acid or a salt thereof and L-phenylalanine R-ester or acid salt thereof, wherein R-ester represents an ester residue of an alcohol having 2 or more carbon atoms or a substituted or unsubstituted phenol, with a microorganism or enzyme-containing fraction of said microorganism capable of forming L-aspartyl-L-phenylalanine ester by the condensation of L-aspartic acid and L-phenylalanine ester and belonging to a genus selected from Corynebacterium, Candida, Escherichia, Flavobacterium, Geotrichum, Micrococcus, Pachysolen, Trichosporon, Xanthomonas, Kluyveromyces, Endomyces, Arthrobacter, Cellulomonas and Brevibacterium.

2. The process of Claim 1 wherein the microorganism is selected from:

| | |
|---|---|
| Corynebacterium sp. | ATCC 21251 |
| Corynebacterium xerosis | ATCC 373 |
| Candida intermedia | FERM-BP508 |
| Escherichia coli | FERM-BP477 |
| Flavobacterium sewanens | FERM-BP476 |
| Geotrichum candidum | IFO 4599 |
| Micrococcus luteus | ATCC 4698 |
| Pachysolen tannophilus | IFO 1007 |
| Trichosporon capitatum | IFO 1197 |
| Xanthomonas campestris | FERM-BP507 |
| Kluyveromyces thermotolerans | IFO 0662 |
| Endomyces ovetencis | IFO 1201 |
| Arthrobacter citreus | ATCC 11624 |
| Cellulomonas flavigena | ATCC 8183 |
| Brevibacterium linens | ATCC 8377 |

7

3. A process of Claim 1 wherein the microorganism is selected from:

| | |
|---|---|
| Corynebacterium sp. | ATCC 21251 |
| Escherichia coli | FERM-BP477 |
| Flavobacterium sewanens | FERM-BP476 |
| Micrococcus luteus | ATCC 4698 |
| Arthrobacter citreus | ATCC 11624 |
| Cellulomonas flavigena | ATCC 8183 |
| Brevibacterium linens | ATCC 8377 |

4. A process of Claim 1 wherein the microorganism is selected from:

| | |
|---|---|
| Escherichia coli | FERM-BP477 |
| Flavobacterium sewanens | FERM-BP476 |
| Arthrobacter citreus | ATCC 11624 |

5. A process of any one of Claims 1 to 4 wherein R is selected from propyl and butyl.

6. A process of any one of Claims 1 to 4 wherein R is selected from isopropyl and n-butyl.

7. The process of any one of the preceding claims wherein the microorganism is cultured under aerobic conditions at pH 4—8 and at a temperature of 25°C—40°C for a time period in the range of 1—10 days.

**Patentansprüche**

1. Verfahren zur Herstellung eines Süßungsmittels, das umfaßt:

In-Kontakt-Bringen von L-Asparaginsäure oder eines ihrer Salze und L-Phenylalanin-R-Ester oder eines seiner sauren Salze, worin R-Ester für einen Ester-Rest eines Alkohols mit 2 oder mehr Kohlenstoffatomen oder ein substituiertes oder unsubstituiertes Phenol steht, in einem wäßrigen Medium mit einem Mikroorganismus oder einer Enzym-haltigen Fraktion des Mikroorganismus, der durch Kondensation von L-Asparaginsäure und L-Phenylalanin-Ester L-Aspartyl-L-Phenylalanin-Ester bilden kann und zu einer Art aus der Gruppe Corynebacterium, Candida, Escherichia, Flavobacterium, Geotrichum, Micrococcus, Pachysolen, Trichosporon, Xanthomonas, Kluyveromyces, Endomyces, Arthrobacter, Cellulomonas und Brevibacterium gehört.

2. Verfahren nach Anspruch 1, worin der Mikroorganismus ausgewählt ist von

| | |
|---|---|
| Corynebacterium sp. | ATCC 21251 |
| Corynebacterium xerosis | ATCC 373 |
| Candida intermedia | FERM-BP508 |
| Escherichia coli | FERM-BP477 |
| Flavobacterium sewanens | FERM-BP476 |
| Geotrichum candidum | IFO 4599 |
| Micrococcus luteus | ATCC 4698 |
| Pachysolen tannophilus | IFO 1007 |
| Trichosporon capitatum | IFO 1197 |
| Xanthomonas campestris | FERM-BP507 |
| Kluyveromyces thermotolerans | IFO 0662 |
| Endomyces ovetencis | IFO 1201 |
| Arthrobacter citreus | ATCC 11624 |
| Cellulomonas flavigena | ATCC 8183 |
| Brevibacterium linens | ATCC 8377. |

3. Verfahren nach Anspruch 1, worin der Mikroorganismus ausgewählt ist von

| | |
|---|---|
| Corynebacterium sp. | ATCC 21251 |
| Escherichia coli | FERM-BP477 |
| Flavobacterium sewanens | FERM-BP476 |
| Micrococcus luteus | ATCC 4698 |
| Arthrobacter citreus | ATCC 11624 |
| Cellulomonas flavigena | ATCC 8183 |
| Brevibacterium linens | ATCC 8377. |

4. Verfahren nach Anspruch 1, worin der Mikroorganismus ausgewählt ist von

| | |
|---|---|
| Escherichia coli | FERM-BP477 |
| Flavobacterium sewanens | FERM-BP476 |
| Arthrobacter citreus | ATCC 11624. |

5. Verfahren nach einem der Ansprüche 1 bis 4, worin R gewählt ist unter Propyl und Butyl.

6. Verfahren nach einem der Ansprüche 1 bis 4, worin R gewählt ist unter Isopropyl und n-Butyl.

7. Verfahren nach einem der vorangehenden Ansprüche, worin der Mikroorganismus unter aeroben Bedingungen bei einem pH-Wert von 4 bis 8 und einer Temperatur von 25°C bis 40°C für eine Zeitdauer im Bereich von 1 bis 10 Tagen gezüchtet wird.

**Revendications**

1. Un procédé de production d'un agent édulcorant, qui comprend la mise en contact dans un milieu aqueux de l'acide L-aspartique ou d'un sel de ce dernier et de l'ester R de la L-phénylalanine ou d'un sel d'acide de ce dernier, où l'ester R représente un résidu ester d'un alcool ayant 2 ou plus de 2 atomes de carbone ou d'un phénol substitué ou non substitué, avec un micro-organisme ou une fraction contenant l'enzyme dudit microorganisme capable de former l'ester de la L-aspartyl-L-phénylalanine par condensation de l'acide L-aspartique et de l'ester de la L-phénylalanine et appartenant au genre choisi parmi les suivants: Corynebacterium, Candida, Escherichia, Flavobacterium, Geotrichum, Micrococcus, Pachysolen, Trichosporon, Xanthomonas, Kluyveromyces, Endomyces, Arthrobacter, Cellulomonas et Brevibacterium.

2. Le procédé selon la revendication 1, selon lequel le micro-organisme est choisi parmi les suivants:

| | |
|---|---|
| Corynebacterium sp. | ATCC 21251 |
| Corynebacterium xerosis | ATCC 373 |
| Candida intermedia | FERM-BP508 |
| Escherichia coli | FERM-BP477 |
| Flavobacterium sewanens | FERM-BP476 |
| Geotrichum candidum | IFO 4599 |
| Micrococcus luteus | ATCC 4698 |
| Pachysolen tannophilus | IFO 1007 |
| Trichosporon capitatum | IFO 1197 |
| Xanthomonas campestris | FERM-BP507 |
| Kluyveromyces thermotolerans | IFO 0662 |
| Endomyces ovetencis | IFO 1201 |
| Arthrobacter citreus | ATCC 11624 |
| Cellulomonas flavigena | ATCC 8183 |
| Brevibacterium linens | ATCC 8377 |

3. Un procédé selon la revendication 1, selon lequel le micro-organisme est choisi parmi les suivants:

| | |
|---|---|
| Corynebacterium sp. | ATCC 21251 |
| Escherichia coli | FERM-BP477 |
| Flavobacterium sewanens | FERM-BP476 |
| Micrococcus luteus | ATCC 4698 |
| Arthrobacter citreus | ATCC 11624 |
| Cellulomonas flavigena | ATCC 8183 |
| Brevibacterium linens | ATCC 8377 |

4. Un procédé selon la revendication 1, selon lequel le micro-organisme est choisi parmi les suivants:

| | |
|---|---|
| Escherichia coli | FERM-BP477 |
| Flavobacterium sewanens | FERM-BP476 |
| Arthrobacter citreus | ATCC 11624 |

5. Un procédé selon l'une quelconque des revendications 1 à 4, selon lequel R est choisi parmi les groupes propyle et butyle.

6. Un procédé selon l'une quelconque des revendications 1 à 4, selon lequel R est choisi parmi les groupes isopropyle et n-butyle.

7. Un procédé selon l'une quelconque des revendications précédentes, selon lequel le micro-organisme est cultivé dans des conditions en aérobie à pH 4—8 et à une température de 25°C—40°C pendant une période de temps comprise dans l'intervalle de 1—10 j.